(19) Europäisches Patentamt
European Patent Office
Office européen des brevets

(11) **EP 1 266 217 B1**

(12) **EUROPÄISCHE PATENTSCHRIFT**

(45) Veröffentlichungstag und Bekanntmachung des Hinweises auf die Patenterteilung:
**20.02.2008 Patentblatt 2008/08**

(21) Anmeldenummer: **01913639.9**

(22) Anmeldetag: **15.02.2001**

(51) Int Cl.:
**G01N 33/28** (2006.01)

(86) Internationale Anmeldenummer:
**PCT/DE2001/000563**

(87) Internationale Veröffentlichungsnummer:
**WO 2001/067096 (13.09.2001 Gazette 2001/37)**

(54) **VERFAHREN ZUR BEURTEILUNG DES VERSCHLEISSES VON MOTORÖL UNTER BERÜCKSICHTIGUNG EINER FRISCHÖLNACHFÜLLUNG**

METHOD FOR ASSESSING THE WEAR OF MOTOR OIL WHILE TAKING A REPLENISHING THAT INVOLVES THE USE OF FRESH OIL INTO ACCOUNT

PROCEDE POUR ESTIMER LE DEGRE D'ALTERATION D'HUILE DE MOTEUR COMPTE TENU DU COMPLEMENT DE NIVEAU EN HUILE NEUVE

(84) Benannte Vertragsstaaten:
**DE FR GB IT**

(30) Priorität: **07.03.2000 DE 10010976**

(43) Veröffentlichungstag der Anmeldung:
**18.12.2002 Patentblatt 2002/51**

(73) Patentinhaber: **ROBERT BOSCH GMBH**
**70442 Stuttgart (DE)**

(72) Erfinder: **JAKOBY, Bernhard**
**72764 Reutlingen (DE)**

(56) Entgegenhaltungen:
**US-A- 5 750 887      US-A- 5 817 928**
**US-A- 6 023 961**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).

**Beschreibung**

Die Erfindung betrifft ein Verfahren zur Beurteilung des Verschleißes von Motoröl unter Berücksichtigung einer Frischölnachfüllung mit den im Oberbegriff des Anspruches 1 genannten Merkmalen.

Stand der Technik

Es sind software-basierte Systeme zur Anzeige eines notwendigen Motorölwechsels bei Kraftfahrzeugen bekannt, von denen einige auf Algorithmen basieren, die Parameter wie etwa die seit dem letzten Ölwechsel zurückgelegte Distanz oder die Häufigkeit von Kaltstarts auswerten. Des Weiteren wird bei anderen bekannten Verfahren auf Sensorsignale zurückgegriffen, die direkt den physikalischen Ölzustand beschreiben, wie beispielsweise die Dielektrizitätszahl des Öls oder, als weit zuverlässigere Größe, die Ölviskosität ($\eta$). Aus der Registrierung der Viskositätsänderung seit dem letzten Motorölwechsel wird dabei ein viskositäts-basiertes Ölwechselkriterium abgeleitet, da in der Regel mit dem Verschleiß des Motoröles ein Viskositätsanstieg verbunden ist. Als Grenzwert kann zum Beispiel im einfachen Fall ein Viskositätswert ($\eta_{max}$) festgelegt, der den Viskositätswert ($\eta_0$) des Frischöls als Referenzwert um nicht mehr als einen bestimmten Prozentsatz übersteigen darf, beispielsweise um nicht mehr als 30%. Die Viskositätsschwelle, ab der ein Motorölwechsel dann erfolgen sollte und die einem Fahrzeugführer angezeigt wird, ist dann beispielsweise gegeben durch die Beziehung:

$$\eta_{max} = k \times \eta_0 \qquad\qquad (1)$$

Der Faktor k definiert den zulässigen Viskositätsanstieg, bei 30% erlaubter Zunahme entspricht k = 1,3.

In US 5750887 wird ein Verfahren zur Schätzung der Restlebensdauer von Motoröl vorgeschlagen bei dem die Viskosität des Öls und verschiedene andere Parameter des Motors wie beispielsweise Motorlaufzeit, startverhalten Drehzahl etc. eingehen.

Problematisch bei diesen und ähnlichen Verfahren zur Beurteilung des Verschleißes von Motoröl anhand von Viskositätsmessungen ist das Nachfüllen von Frischöl einer anderen Sorte oder Viskositätsklasse. Das nachgefüllte Öl weist dann eine andere Frischölviskosität auf, wodurch das Ölwechselkriterium einer gewissen Verzerrung unterworfen wird. So wird z. B. die Nachfüllung eines Motoröles einer höheren Viskositätsklasse eine Alterung vortäuschen, obwohl defacto durch die Nachfüllung eine Verbesserung des Ölzustandes erreicht wird. Umgekehrt suggeriert die Nachfüllung eines niedrigerviskosen Motoröls eine Verbesserung des Ölzustandes in einem Maße, das tatsächlich nicht vorliegt. Die Frischöl-Viskositätswerte für Motoröle aus benachbarten Viskositätsklassen unterscheiden sich typischerweise bereits um 30%.

Vorteile der Erfindung

Das erfindungsgemäße Verfahren bietet gegenüber dem Stand der Technik den Vorteil, dass eine Beurteilung des Verschleißes von Motoröl anhand von Viskositätsmessungen unter Berücksichtigung einer Frischölnachfüllung vorgenommen werden kann, mit dem eine Korrektur des Referenzwertes möglich ist.

Im Einzelnen wird zunächst durch den Ölfüllstands-Sensor die Ölmenge der Nachfüllung von Frischöl detektiert, danach durch den Ölviskositäts-Sensor nach einer Betriebszeit und/oder ab Erreichen einer vorherbestimmten Öltemperatur die Viskosität des Ölgemisches ($\eta_2$) aus Motoröl und Frischöl bestimmt, in der Auswerteelektronik aus der Differenz des alten Ölstandes bzw des Ölvolumens ($V_1$) und des neuen Ölstandes bzw. des Ölvolumens ($V_2$) die Menge ($V_n$) des zugefügten Frischöles und aus der Viskosität ($\eta_1$) des alten Motoröles unmittelbar vor dem Zeitpunkt des Nachfüllens und der Viskosität ($\eta_2$) nach dem Nachfüllen die Viskosität ($\eta_n$) des Frischöls bestimmt und aus dem bekannten Wert der Viskosität ($\eta_0$) des alten Motoröles im Neuzustand, der Menge ($V_1$) des zum Zeitpunkt des Nachfüllens noch vorhandenen Motoröles, der berechneten Viskosität ($\eta_n$) des Frischöles und der berechneten Menge ($V_n$) des Frischöls eine hypothetische Ausgangs-Viskosität ($\eta'_0$) als neuer korrigierter Referenzwert errechnet wird.

Aus diesem korrigierten Referenzwert lässt sich ein neuer Grenzwert ($\eta'_{max}$) für die Viskositätsschwelle des Ölgemisches berechnen, ab dem ein Ölwechsel erfolgen sollte, welcher den ursprünglichen Wert aus (1) ersetzt, beispielsweise durch folgende Rechenregel:

$$\eta'_{max} = k \times \eta'_0 \qquad\qquad (2)$$

Vorteilhafterweise wird zur Bestimmung der Viskosität ($\eta$) des zugefüllten Frischöles und auch zur Bestimmung der hypothetischen Ausgangsviskosität ($\eta'_0$) als neuer korrigierter Referenzwert jeweils eine einfache Mischungsregel verwendet, bei der der gesuchte Wert eine einfache Funktion der bekannten Viskositäten und den verschiedenen Ölmengen ist.

Gemäß einer weiteren bevorzugten Ausführungsform des Verfahrens können von der angeschlossenen Auswerteelektronik weitere Parameter für die Ölalterung bei der Bestimmung des Grenzwertes der maximal zulässigen Viskosität ($\eta'_{max}$) als Viskositätsschwelle berücksichtigt werden, die Temperaturgradienten der Erwärmung des Motoröles, die Anzahl der Kaltstarts oder auch die Länge der zurückgelegten Fahrstrecken bzw. der Betriebszeiten.

Auch die Bestimmung des korrigierten Referenzwertes ($\eta'_0$) lässt sich durch in Betrachtziehung weiterer Abhängigkeiten und Bewertungskriterien weiter präzisieren und sogar als Referenzcharakteristik in der Auswerteelektronik abspeichern. Beispielsweise kann die Temperaturabhängigkeit der Viskosität bei den Auswertungskriterien mit herangezogen gezogen werden.

Zur weiteren Präzisierung des Referenzwertes können weitere bekannte Bewertungsverfahren zur Beurteilung des Verschleißes von Motoröl mit berücksichtigt werden, so dass die Motorölqualität eines Fahrzeuges nie unter einen erforderlichen Wert fallen kann, ohne dass der Fahrzeuglenker auf einen dringenden Motorölwechsel aufmerksam gemacht wird.

Zeichnungen

Nachfolgend wird das Verfahren anhand zweiter Darstellungen näher beschrieben. Es zeigt:

Figur 1: ein Koordinatensystem, welches das abnehmende Ölvolumen über die Betriebszeit oder zurückgelegte Distanz eines Kraftfahrzeuges darstellt und

Figur 2: ein Koordinatensystem, welches die typischerweise ansteigende Viskosität über die Betriebszeit eines Motors oder die zurückgelegte Distanz wiedergibt.

Beschreibung des Ausführungsbeispiels

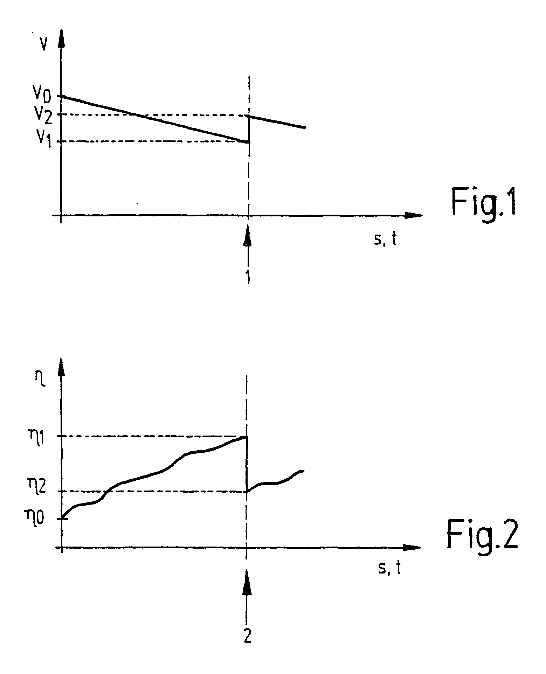

Figur 1 zeigt die typische Veränderung des Ölvolumens ($V_0$) vom Zeitpunkt des letzten Ölwechsels bis zum Zeitpunkt (1) einer Frischölnachfüllung ($V_1$), nach der das Ölvolumen wieder auf einen Wert ($V_2$) ansteigt, wobei die verschiednen Mengen bzw. Ölvolumina in Abhängigkeit vom Füllstand über den Füllstands-Sensor und die Auswerteelektronik bestimmt werden.

Fig. 2 zeigt dagegen das Ansteigen der Viskosität eines Motoröles ausgehend von einer Viskosität ($\eta_0$) im Neuzustand eines Motoröles bis zum Nachfüllzeitpunkt (2) bis auf eine Viskosität ($\eta_1$). Das Nachfüllen von Frischöl führt dann zu einem Sprung auf den Wert der Viskosität ($\eta_2$), wobei dieser die Mischung von bereits teilweise "verbrauchtem" Öl und nachgefülltem Frischöl charakterisiert. Für die Darstellung in der Fig. 2 wurde angenommen, dass der resultierende Viskositätswert ($\eta_2$) bei der Nachfüllung sinkt, was nicht notwendigerweise der Fall sein muss, falls Motoröl einer anderen, höheren Viskositätsklasse als das beim letzten Ölwechsel eingefüllte Öl nachgefüllt wird. Der Viskositätswert ($\eta_2$) wird sich aufgrund unvollständiger Mischung nicht unmittelbar nach dem Nachfüllen, sondern erst nach einiger Betriebszeit des Motors und gründlicher Durchmischung der Motoröle zu einem homogenen Gemisch als stetiger Messwert einstellen.

Die Menge des nachgefüllten Frischöles ($V_n$) wird aus den gemessenen Ölständen bzw. den daraus abgeleiteten Volumina bestimmt:

$$V_n = V_2 - V_1.$$

Zur Bestimmung der a priori unbekannten Viskosität ($\eta_n$) des nachgefüllten Motoröles wird eine Mischungsregel verwendet. Derartige Mischungsregeln werden aufgrund von werkstoffwissenschaftlichen Modellen für die jeweils betrachteten Materialien erstellt und beschreiben die resultierende Viskosität für ein Gemisch aus zwei Flüssigkeiten. Allgemein ergibt sich die Viskosität der Mischung aus dem nachgefüllten Frischöl und dem bereits vorhandenen Öl als Abhängigkeit aus der Viskosität ($\eta_1$) des bereits vorhandenen Öls, dessen Volumenanteil ($V_1$), der Viskosität ($\eta_n$) des nachgefüllten Frischöls sowie dessen Volumen ($V_n$). Ein sehr einfaches Modell für eine solche Mischungsregel geht von der Annahme aus, dass die Viskositäten der gemischten Öle mit den entsprechenden relativen Volumenanteilen gewichtet werden:

$$\eta_2 \; = \; \eta_1 \; (V_1 / (V_1 \; + \; V_n)) \; + \; \eta_n \; (V_n / (V_1 \; + \; V_n))$$

Durch Umformung kann die Viskosität ($\eta_n$) des beigefügten Frischöles aus den bekannten Größen $V_1$, $\eta_1$, $V_n$ und $\eta_2$ berechnet werden. Unter abermaliger Verwendung der Mischungsregel wird die hypothetische Viskosität ($\eta'_0$) ermittelt, die sich bei Mischung der Menge ($V_1$) beim letzten Ölwechsel eingefüllten Frischöls mit der Viskosität ($\eta_0$) und der Menge ($V_n$) des nachgefüllten Fischöls der Viskosität ($\eta_n$) ergeben würde. Aus diesem neuen korrigierten Referenzwert lässt sich der neue Grenzwert $\eta'_{max}$ beispielsweise aus dem Produkt mit dem Faktor k für das Ölgemisch berechnen, der dann den ursprünglichen Wert (1) ersetzt.

Der absolute Grenzwert für die Zulässigkeit einer Viskosität kann jedoch auch allgemeiner als Funktion formuliert werden, in der die Ausgangsviskosität ($\eta_0$) des Frischöls oder die errechnete hypothetische Ausgangsviskosität ($\eta'_0$) des Ölgemisches neben anderen Parametern zur Empfehlung eines Ölwechsels berücksichtigt werden. Die Veränderung der Viskosität gegenüber dem Referenzwert wird im allgemeinen Fall in einen komplexeren Algorithmus einfließen, der noch weitere Kennwerte berücksichtigt.

Das Verfahren zur Beurteilung des Verschleißes von Motoröl anhand von Viskositätsmessungen unter Berücksichtigung einer Frischölnachfüllung erfolgt demnach durch die Charakterisierung des Ölgemisches durch einen das Frischöl repräsentierenden Grenzwert. Bei Ölnachfüllung wird aus der nachgefüllten Ölmenge und der sich ergebenden Mischviskosität ein neuer korrigierter Referenzwert für das resultierende Ölgemisch ermittelt, wobei der partielle Verschleiß des sich bereits im Motor befindlichen Öls berücksichtigt wird. Der Referenzwert kann auch eine Referenzcharakteristik sein, wenn etwa die Temperaturabhängigkeit der Viskosität mit in Betracht gezogen wird. Die viskositäts-basierte Verschleißbewertung kann auch nur einer von mehreren Aspekten eines allgemeineren Bewertungsallgorithmus zur Beurteilung des Motorölzustandes sein.

**Patentansprüche**

1. Verfahren zur Beurteilung des Verschleißes von Motoröl anhand von Viskositätsmessungen unter Berücksichtigung einer Frischölnachfüllung mit Verwendung eines Ölviskositäts-Sensors, eines Ölfüllstands-Sensors und einer angeschlossenen Auswerteelektronik mit Anzeigevorrichtung, bei dem die Alterung des Motoröles und eine daraus resutierende Ölwechselempfehlung durch Bewertung der Veränderung der aktuellen Ölviskosität bei mindestens einer Temperatur gegenüber der Viskosität des beim letzten Ölwechsel eingefüllten Frischöles als Referenzwert ermittelt wird, **dadurch gekennzeichnet, dass**

   - durch den Ölfüllstands-Sensor die Ölmenge der Nachfüllung von Frischöl detektiert,
   - durch den Ölviskositäts-Sensor die Viskosität des Ölgemisches ($\eta_2$) aus gealtertem Motoröl und Frischöl mit der Temperatur bei mindestens einem Temperaturwert bestimmt wird
   - in der Auswerteelektronik aus der Differenz des alten Ölstandes bzw des Ölvolumens ($V_1$) und des neuen Ölstandes bzw. des Ölvolumens ($V_2$) die Menge ($V_n$) des zugefügten Frischöles
   - aus der Viskosität ($\eta_1$) des alten Motoröles unmittelbar vor dem Nachfüllen und der Viskosität ($\eta_2$) unmittelbar nach dem Nachfüllen die Viskosität ($\eta_n$) des Frischöls bestimmt wird und
   - aus dem bekannten Wert der Viskosität ($\eta_0$) des alten Motoröles im Neuzustand, der Menge ($V_1$) des zum Zeitpunkt des Nachfüllens noch vorhandenen Motoröles, der berechneten Viskosität ($\eta_n$) des Frischöles und der berechneten Menge ($V_n$) des Frischöls eine hypothetische Ausgangs-Viskosität ($\eta'_0$) als neuer korrigierter Referenzwert errechnet wird.

2. Verfahren nach Anspruch 1, **dadurch gekennzeichnet, dass** aus der Ausgangsviskosität ($\eta_0$) durch Multiplikation mit einem Faktor (k) ein Grenzwert der Viskosität ($\eta_{max}$) ermittelt wird, dessen Überschreiten durch die aktuell gemessene Viskosität bei einem entsprechenden Temperaturwert die Anzeige des Endes des Ölwechselintervalles durch die Anzeigevorrichtung bewirkt und dass bei Frischölnachfüllung sich der neue Grenzwert ($\eta'_{max}$) aus der errechneten neuen Ausgangs-Viskosität ($\eta'_0$) durch Multiplikation mit dem Faktor (k) des zulässigen Viskositätsanstieges ergibt.

3. Verfahren nach Anspruch 2, **dadurch gekennzeichnet, dass** der Faktor einen Wert größer als 1 aufweist.

4. Verfahren nach einem der beiden vorgenannten Ansprüche, **dadurch gekennzeichnet, dass** zur Bestimmung der Viskosität ($\eta_n$) des Frischöles und/oder zur Bestimmung der hypothetischen Ausgangs-Viskosität ($\eta_0$) des Ölgemisches im Neuzustand als neuer korrigierter Referenzwert eine Mischungsregel verwendet wird.

5. Verfahren nach einem der vorgenannten Ansprüche, **dadurch gekennzeichnet, dass** bei der Bestimmung des Ölwechselintervalles indirekte Einflussfaktoren für die Alterung des Motoröls, berücksichtigt werden.

6. Verfahren nach Anspruch 5, **dadurch gekennzeichnet, dass** als indirekter Einflussfaktor für die Alterung des Motoröls Temperaturgradienten der Ölerwärmung berücksichtigt werden.

7. Verfahren nach Anspruch 5, **dadurch gekennzeichnet, dass** als indirekter Einflussfaktor für die Alterung des Motoröls die Anzahl der gefahrenen Kilometer bzw. die Anzahl der Betriebsstunden seit dem letzten Ölwechsel berücksichtigt werden.

8. Verfahren nach Anspruch 5, **dadurch gekennzeichnet, dass** als indirekter Einflussfaktor für die Alterung des Motoröls Drehzahl / Öltemperaturverläufe berücksichtigt werden.

9. Verfahren nach Anspruch 5, **dadurch gekennzeichnet, dass** als indirekter Einflussfaktor für die Alterung des Motoröls die Dielekrizitätszahl (Permittivität) des Motoröles berücksichtigt wird.

10. Verfahren nach Anspruch 5, **dadurch gekennzeichnet, dass** als indirekter Einflussfaktor für die Alterung des Motoröls die Leitfähigkeit des Motoröles berücksichtigt wird.

11. Verfahren nach Anspruch 5, **dadurch gekennzeichnet, dass** als indirekte Einflussfaktoren für die Alterung des Motoröls Die Häufigkeit von Kaltstarts und die Beladung des Fahrzeuges berücksichtigt werden.

12. Verfahren nach einem der vorgenannten Ansprüche, **dadurch gekennzeichnet, dass** der korrigierte Referenzwert ($\eta'_0$) durch Berücksichtigung zusätzlich gemessener Abhängigkeiten als Referenzcharakteristik ausgebildet ist.

13. Verfahren nach Anspruch 12, **dadurch gekennzeichnet, dass** die Referenzcharakteristik eine Viskositäts / Temperaturcharakteristik ist, wobei nicht gemessene Bereiche durch Inter / Extrapolationsverfahren ergänzt werden.

**Claims**

1. Method for assessing the wear of engine oil by means of viscosity measurements taking into account a fresh oil refill using an oil viscosity sensor, an oil filling level sensor and a connected evaluation electronic system with display device, in which the ageing of the engine oil and a resulting oil change recommendation is determined as a reference value by evaluating the change in the current oil viscosity at at least one temperature compared to the viscosity of the fresh oil which was filled in at the last oil change, **characterized in that**

   - the quantity of oil in the refill of fresh oil is detected by the oil filling level sensor,
   - the viscosity of the oil mixture ($\eta_2$) of aged engine oil and fresh oil is determined by the oil viscosity sensor with the temperature at at least one temperature value,
   - the quantity ($V_n$) of the added fresh oil is determined in the evaluation electronic system from the difference between the old oil level and/or oil volume ($V_1$) and the new oil level and/or oil volume ($V_2$),
   - the viscosity ($\eta_n$) of the fresh oil is determined from the viscosity ($\eta_1$) of the old engine oil directly before the refill and the viscosity ($\eta_2$) directly after the refill, and
   - a hypothetical initial viscosity ($\eta'_0$) is calculated as a new corrected reference value from the known value of the viscosity ($\eta_0$) of the old engine oil in the new state, the quantity ($V_1$) of the still present engine oil at the time of the refill, the calculated viscosity ($\eta_n$) of the fresh oil and the calculated quantity ($V_n$) of the fresh oil.

2. Method according to Claim 1, **characterized in that** a limiting value of the viscosity ($\eta_{max}$) is determined from the initial viscosity ($\eta_0$) by multiplication by a factor ($k$), the upward transgression of which limiting value brings about the display of the end of the oil change interval by the display device by means of the currently measured viscosity at a corresponding temperature value, and **in that** when there is a refill of fresh oil the new limiting value ($\eta'_{max}$) is obtained from the calculated new initial viscosity ($\eta'_0$) by multiplication by the factor ($k$) of the admissible rise in viscosity.

3. Method according to Claim 2, **characterized in that** the factor has a value greater than 1.

4. Method according to one of the two abovementioned claims, **characterized in that** a mixing rule is used to determine

the viscosity ($\eta_n$) of the fresh oil and/or to determine the hypothetical initial viscosity ($\eta_0$) of the oil mixture in the new state as a new corrected reference value.

5. Method according to one of the abovementioned claims, **characterized in that** indirect influencing factors for the ageing of the engine oil are taken into account in the determination of the oil change interval.

6. Method according to Claim 5, **characterized in that** temperature gradients of the heating of the oil are taken into account as an indirect influencing factor for the ageing of the engine oil.

7. Method according to Claim 5, **characterized in that** the number of kilometres travelled and/or the number of operating hours since the last oil change are taken into account as indirect influencing factors for the ageing of the engine oil.

8. Method according to Claim 5, **characterized in that** rotational speed/oil temperature profiles are taken into account as an indirect influencing factor for the ageing of the engine oil.

9. Method according to Claim 5, **characterized in that** the dielectric coefficient (permittivity) of the engine oil is taken into account as an indirect influencing factor for the ageing of the engine oil.

10. Method according to Claim 5, **characterized in that** the conductivity of the engine oil is taken into account as an indirect influencing factor for the ageing of the engine oil.

11. Method according to Claim 5, **characterized in that** the frequency of cold starts and the load on the vehicle are taken into account as indirect influencing factors for the ageing of the engine oil.

12. Method according to one of the abovementioned claims, **characterized in that** the corrected reference value ($\eta'_0$) is formed by taking into account additionally measured dependencies as a reference characteristic.

13. Method according to Claim 12, **characterized in that** the reference characteristic is a viscosity/temperature characteristic, wherein areas which are not measured are supplemented by inter/extrapolation methods.

**Revendications**

1. Procédé d'évaluation du degré d'altération d'une huile de moteur par des mesures de viscosité, tenant compte du complément de niveau d'huile en utilisant un capteur de viscosité d'huile, un capteur de niveau de remplissage d'huile et une électronique d'exploitation reliée à ces capteurs, avec un dispositif d'affichage selon lequel
   on détermine l'altération d'huile du moteur et ainsi d'une recommandation de vidange en exploitant la variation de la viscosité actuelle de l'huile pour au moins une température par rapport à la viscosité de l'huile (9) introduite lors de la dernière vidange comme valeur de référence,
   **caractérisé en ce que**

   - on détecte la quantité d'huile du complément de niveau en huile neuve par le capteur de niveau de remplissage d'huile,
   - avec le capteur de viscosité d'huile on détermine la viscosité du mélange d'huiles ($\eta_2$) à partir de l'huile moteur altérée et de l'huile neuve à la température, pour au moins une valeur de température,
   - dans l'électronique d'exploitation, à partir de la différence entre le niveau d'huile ancien ou le volume d'huile ($V_1$) et le nouveau niveau d'huile ou le volume d'huile ($V_2$) on détermine la quantité ($V_n$) de l'huile neuve ajoutée,
   - à partir de la viscosité ($\eta_1$) de l'huile de moteur altérée, directement après le complément de niveau d'huile et de la viscosité ($\eta_2$), directement après le complément de niveau on détermine la viscosité ($\eta_n$) de l'huile neuve, et
   - à partir de la valeur connue de la viscosité ($\eta_0$) de l'huile de moteur altérée, à l'état neuf, de la quantité ($V_1$) de l'huile de moteur encore présente à l'instant du complément de niveau d'huile, de la viscosité ($\eta_n$) calculée de l'huile neuve et de la quantité calculée ($V_n$) de l'huile neuve, on calcule une viscosité de départ hypothétique ($\eta'_0$) comme nouvelle valeur de référence corrigée.

2. Procédé selon la revendication 1,
   **caractérisé en ce qu'**

à partir de la viscosité de départ ($\eta_0$), par multiplication avec un coefficient (k), on détermine une valeur limite de la viscosité ($\eta_{max}$) dont le dépassement par la viscosité actuelle, mesurée pour une valeur de température correspondante, on affiche la fin de l'intervalle de vidange d'huile par le dispositif d'affichage et **en ce qu'**en cas de complément de niveau d'huile avec de l'huile neuve, on détermine la nouvelle valeur limite ($\eta_{max}$) à partir de la viscosité de départ, nouvelle ($\eta'_0$), calculée par multiplication avec le coefficient (k) de l'augmentation autorisée de la viscosité.

**3.** Procédé selon la revendication 2;
**caractérisé en ce que**
le coefficient a une valeur supérieure à 1.

**4.** Procédé selon l'une des deux revendications précédentes,
**caractérisé en ce que**
on utilise une règle de mélange, pour déterminer la viscosité ($\eta_n$) de l'huile neuve et/ou pour déterminer la viscosité hypothétique de départ ($\eta_0$) du mélange d'huiles à l'état neuf, comme nouvelle valeur de référence corrigée.

**5.** Procédé selon l'une des revendications précédentes,
**caractérisé en ce que**
pour déterminer l'intervalle de vidange d'huile on tient compte des coefficients d'influence indirecte pour l'altération de l'huile de moteur.

**6.** Procédé selon la revendication 5,
**caractérisé en ce que**
comme coefficients d'influence indirecte pour l'altération de l'huile de moteur on tient compte des gradients de température de l'échauffement d'huile.

**7.** Procédé selon la revendication 5,
**caractérisé en ce que**
comme coefficients d'influence indirecte du vieillissement de l'huile de moteur on tient compte du nombre de kilomètres parcourus ou du nombre d'heures de fonctionnement depuis la dernière vidange d'huile.

**8.** Procédé selon la revendication 5,
**caractérisé en ce que**
comme coefficients d'influence indirecte de l'altération de l'huile de moteur on tient compte de la vitesse de rotation/de l'évolution de la température de l'huile.

**9.** Procédé selon la revendication 5,
**caractérisé en ce que**
comme coefficients d'influence indirecte de l'altération de l'huile de moteur on tient compte du coefficient diélectrique (permitativité) de l'huile de moteur.

**10.** Procédé selon la revendication 5,
**caractérisé en ce que**
comme coefficients d'influence indirecte de l'altération de l'huile de moteur on tient compte de la conductivité de l'huile de moteur.

**11.** Procédé selon la revendication 5,
**caractérisé en ce que**
comme coefficients d'influence indirecte de l'altération de l'huile de moteur on tient compte de la fréquence des démarrages à froid et de la charge du véhicule.

**12.** Procédé selon l'une des revendications précédentes,
**caractérisé en ce que**
la valeur de référence corrigée ($\eta_0$) est obtenue en tenant compte de dépendance mesurée en plus comme caractéristique de référence.

**13.** Procédé selon la revendication 12,
**caractérisé en ce que**
la caractéristique de référence est une caractéristique viscosité/température, et les plages non mesurées sont complétées par des procédés d'interpolation/extrapolation.

Fig.1

Fig.2

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- US 5750887 A